(19) Europäisches Patentamt
European Patent Office
Office européen des brevets

(11) Veröffentlichungsnummer : **0 395 991 B1**

(12) # EUROPÄISCHE PATENTSCHRIFT

(45) Veröffentlichungstag der Patentschrift : **23.06.93 Patentblatt 93/25**

(51) Int. Cl.$^5$ : **C07C 225/22,** C07C 255/24, C07D 209/82

(21) Anmeldenummer : **90107723.0**

(22) Anmeldetag : **24.04.90**

(54) **3-Aminophenylpentan-1,5-dione.**

(30) Priorität : **29.04.89 DE 3914379**

(43) Veröffentlichungstag der Anmeldung : **07.11.90 Patentblatt 90/45**

(45) Bekanntmachung des Hinweises auf die Patenterteilung : **23.06.93 Patentblatt 93/25**

(84) Benannte Vertragsstaaten : **CH DE FR GB IT LI**

(56) Entgegenhaltungen :
**EP-A- 0 315 491**
**CHEMISCHE BERICHTE, 90. Jahrgan, 1957, Weinheim; A. TREIBS et al.: "Ueber einen Chinopyrilium Farbstoff", Seiten 789-793**

(73) Patentinhaber : **BASF Aktiengesellschaft**
**Carl-Bosch-Strasse 38**
**W-6700 Ludwigshafen (DE)**

(72) Erfinder : **Raulfs, Friedrich-Wilhelm, Dr.**
**Brandenburger Str. 6**
**W-6703 Limburgerhof (DE)**
Erfinder : **Mayer, Udo, Dr.**
**Max-Slevogt-Strasse 27**
**W-6710 Frankenthal (DE)**
Erfinder : **Oberlinner, Andreas, Dr.**
**Im Zinkig 108**
**W-6700 Ludwigshafen (DE)**

**EP 0 395 991 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 3-Aminophenylpentan-1,5-dione der Formel I

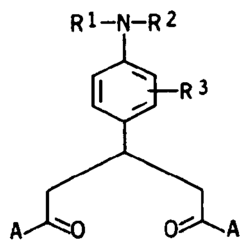

(I),

in der

$R^1$ und $R^2$ gleich oder verschieden sind und unabhängig voneinander jeweils Wasserstoff, $C_1$-$C_8$-Alkyl, $C_4$-$C_8$-Cycloalkyl, Cyano-$C_2$-$C_4$-alkyl, Hydroxy-$C_2$-$C_4$-alkyl, Chlor-$C_2$-$C_4$-alkyl, gegebenenfalls durch Chlor substituiertes Phenyl-$C_1$-$C_2$-alkyl oder gegebenenfalls durch Chlor oder Methyl substituiertes Phenyl oder $R^1$ und $R^2$ zusammen mit dem sie verbindenden Stickstoffatom einen 5- oder 6-gliedrigen gesättigten heterocyclischen Rest, der weitere Heteroatome enthalten kann,

$R^3$ Wasserstoff, Fluor, Chlor, $C_1$-$C_2$-Alkyl oder $C_1$-$C_5$-Alkoxy oder, wenn $R^3$ orthoständig zum Rest -$NR^1R^2$ ist, $R^3$ und $R^2$ zusammen auch $C_2$-$C_4$-Alkylen, das durch 1 bis 3 $C_1$-$C_4$-Alkylreste substituiert sein kann, oder 1,2-Phenylen und

A Naphthyl oder einen Rest der Formel II

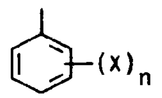

(II),

in der

X für Wasserstoff, Fluor, Chlor, Brom, $C_1$-$C_5$-Alkyl, Hydroxy, $C_1$-$C_5$-Alkoxy, Phenoxy, Phenyl-$C_1$-$C_2$-alkoxy, Cyano oder Phenyl und

n für 1 oder 2 stehen, bedeuten,

mit der Maßgabe, daß $R^1$ und $R^2$ nicht gleichzeitig für Methyl stehen, wenn A die Bedeutung von Phenyl besitzt.

Aus Chem. Ber. Band 90, Seite 789 bis 793, 1957, ist die Herstellung von 3-(p-Dimethylaminophenyl)-1,5-diphenylpentan-1,5-dion bekannt. Diese Verbindung wird dort für die Herstellung eines Pyridins verwendet.

Aufgabe der vorliegenden Erfindung war es, neue 3-Aminophenylpentan-1,5-dione bereitzustellen, die sich in vorteilhafter Weise für die Herstellung von Triphenylcyclopentadienen oder deren Salze eignen.

Demgemäß wurden die eingangs näher bezeichneten 3-Aminophenylpentan-1,5-dione der Formel I gefunden.

Alle in der obengenannten Formel I auftretenden Alkyl- und Alkylengruppen können sowohl geradkettig als auch verzweigt sein.

Reste $R^1$, $R^2$ und X sind z.B. Methyl, Ethyl, Propyl, Isopropyl, Butyl, Isobutyl, sec-Butyl, tert-Butyl, Pentyl, Isopentyl, Neopentyl oder tert-Pentyl.

Reste $R^1$ und $R^2$ sind weiterhin z.B. Hexyl, Heptyl, Octyl, 2-Ethylhexyl, Cyclobutyl, Cyclopentyl, Methylcyclopentyl, Cyclohexyl, Methylcyclohexyl, Cycloheptyl, Cyclooctyl, 2-Cyanoethyl, 2- oder 3-Cyanopropyl, 2-Hydroxyethyl, 2- oder 3-Hydroxypropyl, 2- oder 4-Hydroxybutyl, 2-Chlorethyl, 2- oder 3-Chlorpropyl, 2- oder 4-Chlorbutyl, Benzyl, 1- oder 2-Phenylethyl oder 4-Chlorbenzyl.

$R^1$ und $R^2$ können auch zusammen mit dem sie verbindenden Stickstoffatom einen 5- oder 6-gliedrigen gesättigten heterocyclischen Rest, der weitere Heteroatome, z.B. Stickstoff, Sauerstoff oder Schwefel, enthalten kann, bedeuten. Beispielhaft sind die Reste Pyrrolidino, Piperidino, Morpholino, Thiomorpholino, Thiomorpholino-S,S-dioxid, Piperazino oder N-($C_1$-$C_4$-Alkyl)piperazino, wie N-Methyl- oder N-Ethylpiperazino, zu nennen.

X steht weiterhin, wie auch $R^3$, z.B. für Methoxy, Ethoxy, Propoxy, Isopropoxy, Butoxy, Isobutoxy, sec-Butoxy, Pentyloxy, Isopentyloxy oder Neopentyloxy.

$R^3$ ist weiterhin z.B. Methyl oder Ethyl, oder wenn $R^3$ orthoständig zum Rest -$NR^1R^2$ ist, mit $R^2$ zusammen z.B. Ethylen, 1,2- oder 1,3-Propylen, 1,2-, 1,3-, 2,3- oder 1,4-Butylen oder 1,1,3-Trimethyl-1,3-propylen.

Der Rest A ist beispielsweise Phenyl, 4-Chlorphenyl, 3,4-Dichlorphenyl, 4-Fluorphenyl, 3,4-Difluorphenyl, 4-Methylphenyl, 3-Methylphenyl, 3,4-Dimethylphenyl, 2,5-Dimethylphenyl, 4-Ethylphenyl, 4-Propylphenyl, 4-Isopropylphenyl, 4-Butylphenyl, 4-tert-Butylphenyl, 4-Hydroxyphenyl, 4-Methoxyphenyl, 3-Methoxyphenyl, 2-Methoxyphenyl, 2,4-Dimethoxyphenyl, 2,5-Dimethoxyphenyl, 3,4-Dimethoxyphenyl, 3,5-Dimethoxyphenyl, 4-Ethoxyphenyl, 4-Isopropoxyphenyl, 4-Butoxyphenyl, 4-tert-Butoxyphenyl, 4-Phenoxyphenyl, 4-(2-Phenylethoxy)phenyl, 4-Cyanophenyl, 4-Phenylphenyl, Naphth-1-yl oder Naphth-2-yl.

Wenn $R^1$ und $R^2$ in Formel I für $C_1$-$C_8$-Alkyl stehen, sind solche Verbindungen bevorzugt, in denen $R^1$ unverzweigtes $C_1$-$C_8$-Alkyl und $R^2$ unverzweigtes oder verzweigtes $C_1$-$C_8$-Alkyl bedeuten.

Weiterhin bevorzugt sind 3-Aminophenylpentan-1,5-dione der Formel I, in der $R^3$ Wasserstoff, Methyl oder Methoxy bedeutet.

Besonders bevorzugt sind 3-Aminophenylpentan-1,5-dione der Formel I, in der $R^1$ und $R^2$ unabhängig voneinander unverzweigtes $C_1$-$C_4$-Alkyl, Cyanoethyl oder Benzyl und $R^3$ Wasserstoff bedeuten.

Weiterhin besonders bevorzugt sind 3-Aminophenylpentan-1,5-dione der Formel I, in der A Naphth-1-yl, Naphth-2-yl oder einen Rest der Formel II

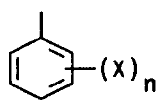

(II),

in der

X für Fluor, Chlor, Brom, $C_1$-$C_5$-Alkyl, Hydroxy, $C_1$-$C_5$-Alkoxy, Phenoxy, Phenyl-$C_1$-$C_2$-alkoxy, Cyano oder Phenyl und

n für 1 oder 2 stehen,

bedeutet.

Insbesondere hervorzuheben sind 3-Aminophenylpentan-1,5-dione der Formel I, in der A Naphth-1-yl, Naphth-2-yl oder einen Rest der Formel IIa

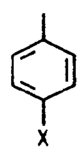

(IIa),

in der

X für Fluor, Chlor, $C_1$-$C_5$-Alkyl, $C_1$-$C_5$-Alkoxy, Cyano oder Phenyl steht, bedeutet.

Weiterhin sind insbesonders hervorzuheben 3-Aminophenylpentan-1,5-dione der Formel I, in der $R^1$ und $R^2$ unabhängig voneinander unverzweigtes $C_1$-$C_4$-Alkyl, Cyanoethyl oder Benzyl und A jeweils 4-Chlorphenyl bedeuten.

Weiterhin sind insbesondere hervorzuheben 3-Aminophenylpentan-1,5-dione der Formel I, in der $R^1$ und $R^2$ jeweils Methyl bedeuten.

Besondere Bedeutung kommt auch den 3-Aminophenylpentan-1,5-dionen der Formel I zu, in der A für Phenyl, 4-Chlorphenyl, 4-Fluorphenyl, 4-Methylphenyl, 4-Methoxyphenyl, Naphth-1-yl oder Naphth-2-yl steht.

Die erfindungsgemäßen 3-Aminophenylpentan-1,5-dione können nach an sich bekannten Methoden, wie z.B. in Chem.Ber. Band 90, Seiten 789 bis 793, 1957, beschrieben, durch alkalische Kondensation eines Aldehyds der Formel III mit überschüssigem Arylketon der Formel IV (im allgemeinen 1,5 bis 4 Mol IV je Mol III), wobei $R^1$, $R^2$, $R^3$ und A jeweils die obengenannte Bedeutung besitzen, gemäß folgendem Schema

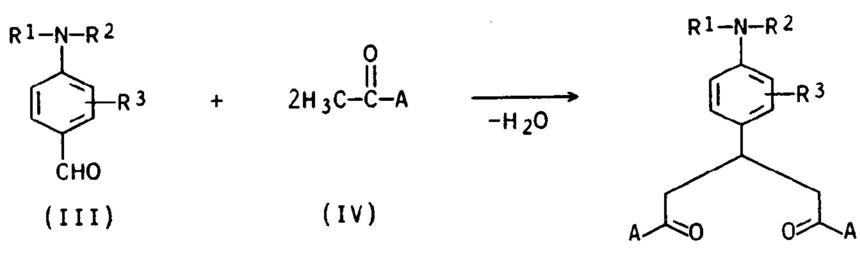


(III) + (IV) → (I)

erhalten werden. Die Umsetzung wird vorteilhaft in Gegenwart eines Lösungsmittels, z.B. eines Alkohols, wie

Methanol, Ethanol, Propanol, Isopropanol, Butanol, Isobutanol, Methylglykol oder 2-Methoxypropanol, oder deren Gemischen mit Wasser vorgenommen.

Alkalihydroxide, wie Natrium- oder Kaliumhydroxid oder Alkalialkoholate, wie Natrium- oder Kaliummethanolat oder -ethanolat, die vorzugsweise in 2-molarem Überschuß, bezogen auf den Aldehyd III, angewendet werden, eignen sich dabei besonders gut als Kondensationsmittel.

Nach einer anfänglich exothermen Reaktion liegt der geeignete Temperaturbereich für die Umsetzung bei einer Temperatur von 20 bis 60°C. Weitere Einzelheiten der Herstellung und der Isolierung der 1,5-Diketone können den Beispielen entnommen werden.

Die erfindungsgemäßen 3-Aminophenylpentan-1,5-dione sind, wie oben bereits erwähnt, wertvolle Zwischenprodukte für die Synthese von Triphenylcyclopentadien-Farbstoffen oder deren Salzen.

Solche Farbstoffe, die die Formel V oder VI

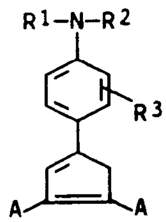

(V)

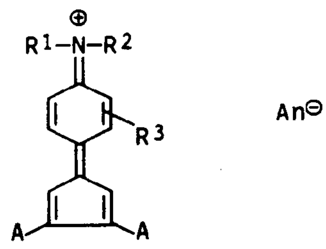

(VI)

aufweisen, wobei $R^1$, $R^2$, $R^3$ und A jeweils die obengenannte Bedeutung besitzen und $An^{\ominus}$ ein Anion bedeutet, sind aus den 3-Aminophenylpentan-1,5-dionen I z.B. dadurch erhältlich, daß man das Dion I mit Zink in Eisessig behandelt (siehe z.B. Bull. Soc. Chim. France 1970, Seiten 252 bis 255), dann das als Zwischenprodukt entstehende Dihydroxycyclopentanderivat mittels Säure (z.B. Salzsäure oder Schwefelsäure) in das Cyclopentadienderivat V überführt, das anschließend durch Umsetzung mit einem Oxidationsmittel (z.B. Halogen, wie Chlor, Brom oder Iod, N-Brom- oder N-Chlorsuccinimid, Bleitetraacetat oder Eisen(III)chlorid) in den ionischen Farbstoff VI umgewandelt werden kann.

Die Farbstoffe der Formel V eignen sich zum Färben von Polyester- oder Polyamidfasern, diejenigen der Formel VI zum Färben von Polyacrylnitrilfasern.

Die folgenden Beispiele sollen die Erfindung näher erläutern.

A) 3-Aminophenylpentan-1,5-dione

Beispiel 1

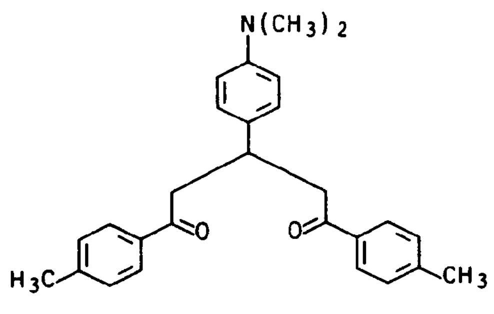

Zu 14,9 g p-Dimethylaminobenzaldehyd und 28,9 g p-Methylacetophenon in 250 ml Isopropanol wurden unter Kühlung 18 g 50 gew.%ige Natronlauge zugetropft. Das Reaktionsgemisch wurde 2 Stunden bei Raumtemperatur und anschließend weitere 4,5 Stunden bei 40°C gerührt, danach wurde es auf 20°C abgekühlt und filtriert. Der Rückstand wurde zunächst bis zu einem schwach gelb gefärbten Ablauf mit Isopropanol und danach mit Wasser neutral gewaschen. Nach dem Trocknen wurden 27,3 g 3-(p-Dimethylaminophenyl)-1,5-bis(4-methylphenyl)pentan-1,5-dion isoliert. Die farblosen Kristalle schmelzen bei 125 bis 126°C.

Beispiel 2

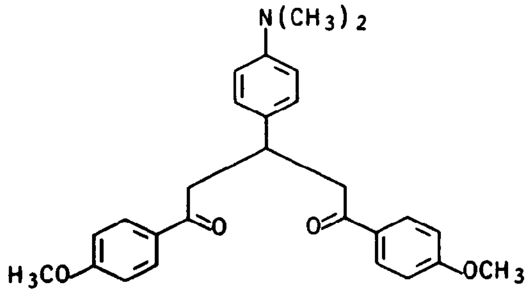

7,2 g Kaliumhydroxid wurden in 150 ml Ethanol gelöst. Nach Zugabe von 14,9 g p-Dimethylaminobenzaldehyd und 28,9 g p-Methoxyacetophenon wurde 2 Stunden bei 25°C, 5 Stunden bei 40°C ud weitere 7 Stunden unter Rückfluß erhitzt. Zur Aufarbeitung wurde das Reaktionsgemisch auf 1 l Wasser und 200 ml Ethylacetat gegeben und mit Eisessig ein pH-Wert von 5 eingestellt. Die organische Phase wurde abgetrennt und zur Klärung filtriert. Ethylacetat wurde im Wasserstrahlvakuum abdestilliert und der verbleibende Rückstand chromatographisch an Kieselgel (Eluens: Toluol/Ethylacetat, 2:1, v/v) getrennt. Nach Umkristallisation aus Cyclohexan wurden 2 g farblose Kristalle von 3-(p-Dimethylaminophenyl)-1,5-bis(p-methoxyphenyl)pentan-1,5-dion isoliert (Schmp.: 70 bis 72°C).

Beispiel 3

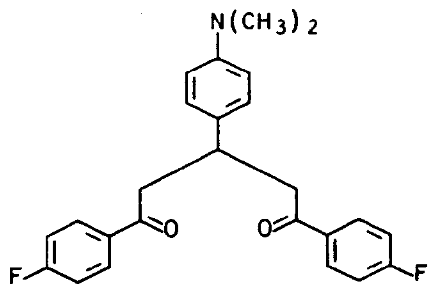

9 g Natriumhydroxid wurden in 150 ml Isopropanol gelöst. Bei 20°C wurden 14,9 g p-Dimethylaminobenzaldehyd und 29,7 g p-Fluoracetophenon hinzugegeben. Nach 2-stündigem Rühren bei 25°C wurde der Niederschlag abfiltriert und das Filtrat langsam eingeengt. Es fielen 9,31 g 3-(p-N,N-Dimethylaminophenyl)-1,5-bis(p-fluorphenyl)pentan-1,5-dion aus, das bei 92°C schmilzt.

Beispiel 4

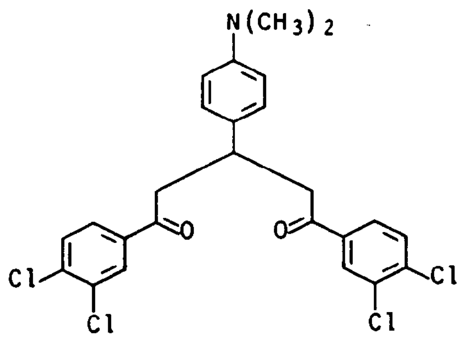

Man verfuhr analog Beispiel 1, jedoch wurden anstelle von p-Methylacetophenon 23,6 g 3,4-Dichloracetophenon verwendet und mit 7,5 g p-Dimethylaminobenzaldehyd zur Reaktion gebracht. Es wurden 18 g 3-(p-N,N-Dimethylaminophenyl)-1,5-bis(3',4'-dichlorphenyl)pentan-1,5-dion erhalten. Die Substanz schmilzt bei 105 bis 107°C.

Analog Beispiel 1 wurden Ketone der Formel A-CO-$CH_3$ mit p-Dimethylaminobenzaldehyd kondensiert, wobei die in der folgenden Tabelle 1 aufgeführten Ketone der Formel

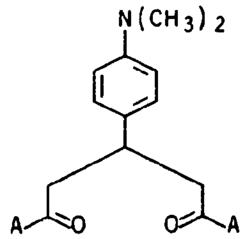

erhalten wurden.

Tabelle 1

| Beispiel Nr. | A | Menge an Keton [g] | Ausbeute [g] | Schmelzbereich [°C] |
|---|---|---|---|---|
| 5 | 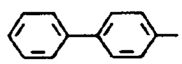 | 49,0 | 45,2 | 194-200 |
| 6 | 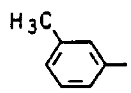 | 16,8 | 12,1 | 85- 87 |
| 7 | 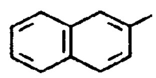 | 21,3 | 15,9 | 129-130 |
| 8 | 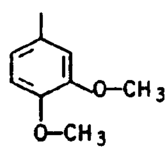 | 45,0 | 29,4 | 133-137 |
| 9 | 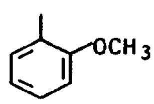 | 18,8 | 9 | 109-111 |
| 10 | 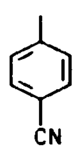 | 9,1 | 3,6 | 226-228 |

Beispiel 11

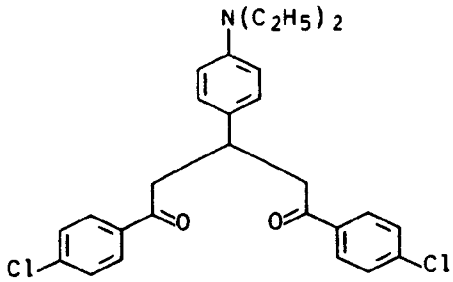

Zu 17,7 g p-Diethylaminobenzaldehyd und 33,3 g p-Chloracetophenon in 150 ml Isopropanol wurden bei 20°C 18 g 50 gew.%ige Natronlauge hinzugetropft. Es wurde 2 Stunden bei 20°C und 5 Stunden bei 40°C nachgerührt. Der abfiltrierte Rückstand wurde zunächst mit Methanol bis zu einem schwach gelb gefärbten Ablauf und dann mit Wasser neutral gewaschen. Nach dem Trocknen wurden 38,6 g 3-(p-N,N-Diethylaminophenyl)-1,5-bis(p-chlorphenyl)pentan-1,5-dion erhalten. Die gelblichen Kristalle schmelzen bei 140°C.

Analog Beispiel 2 wurde 4-Chloracetophenon mit Aldehyden der Formel B-CHO zu den in der folgenden Tabelle 2 aufgeführten 1,5-Diketonen der Formel

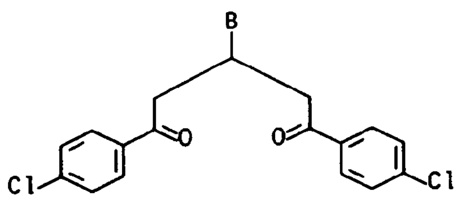

kondensiert.

Tabelle 2

| Beispiel Nr. | B | Menge an Aldehyd [g] | Ausbeute [g] | Schmelzbereich [°C] |
|---|---|---|---|---|
| 12 | 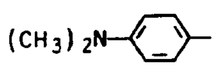 | 14,9 | 38,3 | 103-104 |
| 13 | 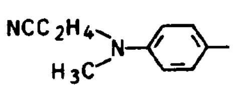 | 18,8 | 13,4 | 89- 96 |
| 14 | 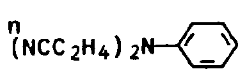 | 21,1 | 22,3 | 118-122 |

Tabelle 2 (Fortsetzung)

| Beispiel Nr. | B | Menge an Aldehyd [g] | Ausbeute [g] | Schmelzbereich [°C] |
|---|---|---|---|---|
| 15 | 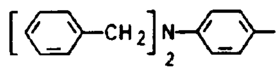 | 15,1 | 17,2 | 93-100 |
| 16 | 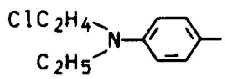 | 10,6 | | |
| 17 | 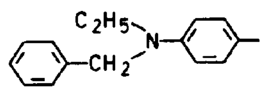 | 12,0 | 12,8 | 112-116 |
| 18 | 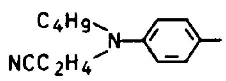 | 17 | 24,7 | 113 |
| 19 | 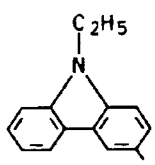 | 11,2 | 22,2 | 103 |

Beispiel 20

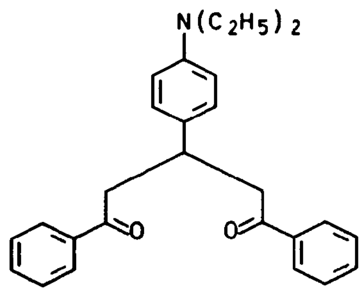

Zu 17,7 g p-Diethylaminobenzaldehyd und 26,4 g Acetophenon in 150 ml Ethanol wurden bei 20°C 18 g 50 gew.%ige Natronlauge hinzugetropft. Es wurde 2 Stunden bei 20°C und 5 Stunden bei 50°C nachgerührt. Der ausgefallene Rückstand wurde abfiltriert, mit Ethanol bis zu einem nur noch schwach gelblichen Rückstand und anschließend mit Wasser neutral gewaschen. Es wurden 33,6 g 3-(p-N,N-Diethylaminophenyl)-1,5-diphenylpentan-1,5-dion erhalten. Die fast farblosen Kristalle schmelzen bei 110°C.

Analog Beispiel 20 wurden die in der folgenden Tabelle 3 aufgeführten Verbindungen der Formel

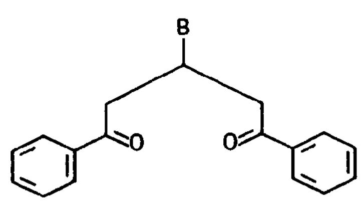

erhalten.

Tabelle 3

| Beispiel Nr. | B | Menge an Aldehyd [g] | Ausbeute [g] | Schmelzbereich [°C] |
|---|---|---|---|---|
| 21 | 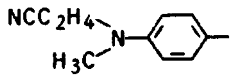 | 9,4 | 8,0 | 110 |
| 22 | 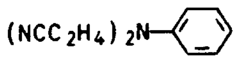 | 11,4 | 16,3 | 115 |
| 23 | 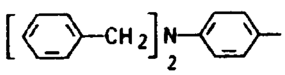 | 15,1 | 14,6 | 139-141 |
| 24 | 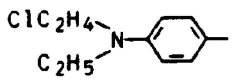 | 10,6 | 11,4 | 94- 95 |
| 25 | 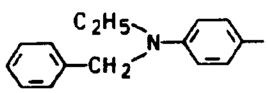 | 12,0 | 19,3 | 124 |
| 26 | 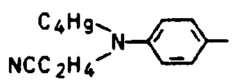 | 18,0 | 14,1 | 93- 98 |
| 27 | 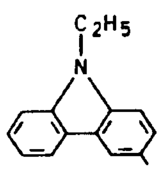 | 19,4 | 13,9 | 78- 84 |

B) Herstellung der Triphenylcyclopentadiene

Beispiel 28

a) Zu 44 g 1,5-Bis(p-chlorphenyl)-3-(p-N,N-dimethylaminophenyl)pentan-1,5-dion (Beispiel 12) in 1000 ml Eisessig wurden innerhalb von 2 Stunden 100 g Zinkpulver bei 80 bis 87°C portionsweise hinzugegeben. Es wurde noch weitere 1,5 Stunden bei 87°C nachgerührt. Das nicht umgesetzte Zink wurde abfiltriert und Eisessig vom verbleibenden Reaktionsgemisch unter vermindertem Druck abdestilliert. Danach wurde mit 500 ml Wasser verdünnt und mit konzentrierter wäßriger Ammoniaklösung ein pH-Wert von 4,2 eingestellt. Das ausgefallene Rohprodukt wurde abgesaugt, unter vermindertem Druck getrocknet und aus Xylol umkristallisiert. Ausbeute: 27,2 g Produkt der Formel

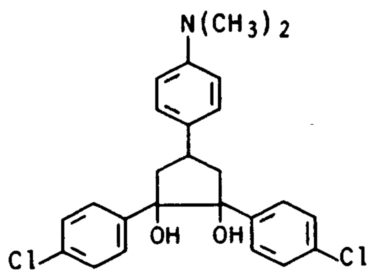

(Schmp.: 114 bis 118°C)

b) Das aus a) resultierende Produkt wurde in 900 ml 38 gew.%ige Salzsäure eingetragen und 4 Stunden bei Raumtemperatur gerührt. Die Suspension wurde mit 1,8 l Wasser verdünnt und filtriert. Der Rückstand wurde in 500 ml Methanol aufgenommen und mit konzentrierter wäßriger Ammoniaklösung ein pH-Wert von 8 eingestellt. Die resultierenden gelben Kristalle wurden aus Ethanol umkristallisiert. Ausbeute: 16,2 g Produkt der Formel

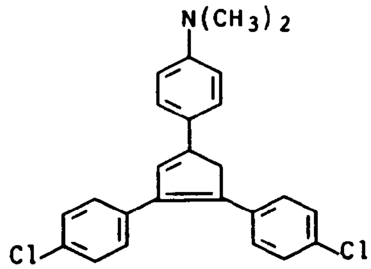

(Schmp.: 138 bis 139°C)

Mit diesem Produkt wurde Polyestergewebe gefärbt (0,6 Gew.% Farbstoff, bezogen auf das Gewebe; Temperatur des Färbebads: 130°C; Färbedauer: 60 Minuten). Man erhielt Ausfärbungen in gelben Farbtönen.

c) Zu einer Lösung des unter b) erhaltenen Produkts in 50 ml Ameisensäureethylester wurden 27 g N-Bromsuccinimid während 5 Minuten bei 50°C portionsweise hinzugefügt. Nach 5minütigem Rühren unter Rückfluß wurde die überstehende Lösunng abdekantiert und der Rückstand mit 50 ml Ameisensäureethylester ausgekocht. Nach dem Abdekantieren wurde der Rückstad 64 Stunden in Ethylacetat gerührt, abfiltriert und getrocknet. Man erhielt 4,5 g Produkt der Formel

($\lambda_{max}$: 557 nm)

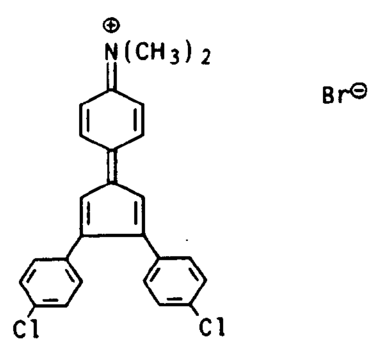

Mit diesem Produkt wurde Polyacrylnitrilgewebe gefärbt (0,6 Gew.% Farbstoff, bezogen auf das Gewebe; Temperatur des Färbebads: 100°C; Färbedauer: 90 Minuten). Man erhielt Ausfärbungen in violetten Farbtönen.

## Patentansprüche

**1.** 3-Aminophenylpentan-1,5-dione der Formel I

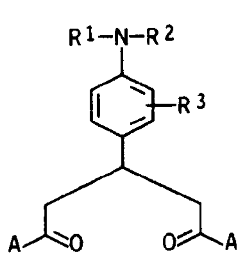

(I),

in der

| | |
|---|---|
| $R^1$ und $R^2$ | gleich oder verschieden sind und unabhängig voneinander jeweils Wasserstoff, $C_1$-$C_8$-Alkyl, $C_4$-$C_8$-Cycloalkyl, Cyano-$C_2$-$C_4$-alkyl, Hydroxy-$C_2$-$C_4$-alkyl, Chlor-$C_2$-$C_4$-alkyl, gegebenenfalls durch Chlor substituiertes Phenyl-$C_1$-$C_2$-alkyl oder gegebenenfalls durch Chlor oder Methyl substituiertes Phenyl oder $R^1$ und $R^2$ zusammen mit dem sie verbindenden Stickstoffatom einen 5- oder 6-gliedrigen gesättigten heterocyclischen Rest, der weitere Heteroatome enthalten kann, |
| $R^3$ | Wasserstoff, Fluor, Chlor, $C_1$-$C_2$-Alkyl oder $C_1$-$C_5$-Alkoxy oder, wenn $R^3$ orthoständig zum Rest $-NR^1R^2$ ist, $R^3$ und $R^2$ zusammen auch $C_2$-$C_4$-Alkylen, das durch 1 bis 3 $C_1$-$C_4$-Alkylreste substituiert sein kann, oder 1,2-Phenylen und |
| A | Naphthyl oder einen Rest der Formel II |

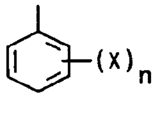

(II),

in der

X für Wasserstoff, Fluor, Chlor, Brom, $C_1$-$C_5$-Alkyl, Hydroxy, $C_1$-$C_5$-Alkoxy, Phenoxy, Phenyl-$C_1$-$C_2$-alkoxy, Cyano oder Phenyl und

n für 1 oder 2 stehen, bedeuten,

mit der Maßgabe, daß $R^1$ und $R^2$ nicht gleichzeitig für Methyl stehen, wenn A die Bedeutung von Phenyl besitzt.

2. 3-Aminophenylpentan-1,5-dione gemäß Anspruch 1, dadurch gekennzeichnet, daß A Naphth-1-yl, Naphth-2-yl oder einen Rest der Formel II

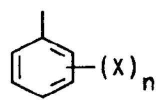

(II),

in der

X für Fluor, Chlor, Brom, $C_1$-$C_5$-Alkyl, Hydroxy, $C_1$-$C_5$-Alkoxy, Phenoxy, Phenyl-$C_1$-$C_2$-alkoxy, Cyano oder Phenyl und

n für 1 oder 2 stehen,

bedeutet.

3. 3-Aminophenylpentan-1,5-dione gemäß Anspruch 1, dadurch gekennzeichnet, daß $R^1$ und $R^2$ unabhängig voneinander unverzweigtes $C_1$-$C_4$-Alkyl, Cyanoethyl oder Benzyl und $R^3$ Wasserstoff bedeuten.

4. 3-Aminophenylpentan-1,5-dione gemäß Anspruch 1, dadurch gekennzeichnet, daß A Naphth-1-yl, Naphth-2-yl oder einen Rest der Formel IIa

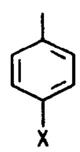

(IIa),

in der

X für Fluor, Chlor, $C_1$-$C_5$-Alkyl, $C_1$-$C_5$-Alkoxy, Cyano oder Phenyl steht,

bedeutet.

5. 3-Aminophenylpentan-1,5-dione gemäß Anspruch 1, dadurch gekennzeichnet, daß $R^1$ und $R^2$ unabhängig voneinander unverzweigtes $C_1$-$C_4$-Alkyl, Cyanoethyl oder Benzyl und A jeweils 4-Chlorphenyl bedeuten.

6. 3-Aminophenylpentan-1,5-dione gemäß Anspruch 1, dadurch gekennzeichnet, daß $R^1$ und $R^2$ jeweils Me-

thyl bedeuten.

## Claims

1. A 3-aminophenylpentane-1,5-dione of the formula I

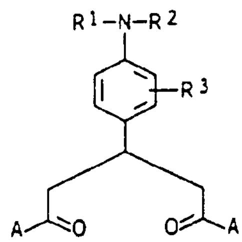

(I)

where

$R^1$ and $R^2$ are identical or different and, independently of one another, are each hydrogen, $C_1$-$C_8$-alkyl, $C_4$-$C_8$-cycloalkyl, cyano-$C_2$-$C_4$-alkyl, hydroxy-$C_2$-$C_4$-alkyl, chloro-$C_2$-$C_4$-alkyl, phenyl-$C_1$-$C_2$-alkyl which can be substituted by chlorine, or phenyl which can be substituted by chlorine or methyl, or $R^1$ and $R^2$ form, together with the nitrogen to which they are bonded, a 5- or 6-membered saturated heterocyclic radical which can contain further hetero atoms,

$R^3$ is hydrogen, fluorine, chlorine, $C_1$-$C_2$-alkyl or $C_1$-$C_5$-alkoxy or, if $R^3$ is ortho to $-NR^1R^2$, $R^3$ and $R^2$ together form $C_2$-$C_4$-alkylene which can be substituted by 1 to 3 $C_1$-$C_4$-alkyls, or 1,2-phenylene and

A is naphthyl or a radical of the formula II

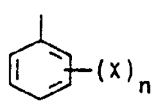

(II)

where

X is hydrogen, fluorine, chlorine, bromine, $C_1$-$C_5$-alkyl, hydroxyl, $C_1$-$C_5$-alkoxy, phenoxy, phenyl-$C_1$-$C_2$-alkoxy, cyano or phenyl and

n is 1 or 2,

with the proviso that $R^1$ and $R^2$ are not both methyl when A is phenyl.

2. A 3-aminophenylpentane-1,5-dione as claimed in claim 1, wherein A is 1-naphthyl, 2-naphthyl or a radical of the formula II

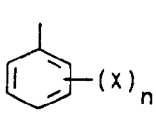

(II)

where

X is fluorine, chlorine, bromine, $C_1$-$C_5$-alkyl, hydroxyl, $C_1$-$C_5$-alkoxy, phenoxy, phenyl-$C_1$-$C_2$-alkoxy, cyano or phenyl and

n is 1 or 2.

3. A 3-aminophenylpentane-1,5-dione as claimed in claim 1, wherein $R^1$ and $R^2$ are, independently of one another, unbranched $C_1$-$C_4$-alkyl, cyanoethyl or benzyl, and $R^3$ is hydrogen.

4. A 3-aminophenylpentane-1,5-dione as claimed in claim 1, wherein A is 1-naphthyl, 2-naphthyl or a radical of the formula IIa

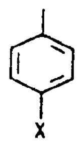

(IIa)

where

X is fluorine, chlorine, $C_1$-$C_5$-alkyl, $C_1$-$C_5$-alkoxy, cyano or phenyl.

5. A 3-aminophenylpentane-1,5-dione as claimed in claim 1, wherein $R^1$ and $R^2$ are, independently of one another, unbranched $C_1$-$C_4$-alkyl, cyanoethyl or benzyl and A is 4-chlorophenyl.

6. A 3-aminophenylpentane-1,5-dione as claimed in claim 1, wherein $R^1$ and $R^2$ are each methyl.

## Revendications

1. 3-Aminophénylpentane-1,5-diones de formule I

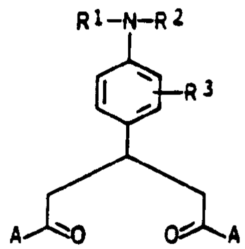

(I),

dans laquelle

$R^1$ et $R^2$ sont identiques ou différents et représentent chacun, indépendamment l'un de l'autre, un atome d'hydrogène ou un reste alkyle en $C_1$-$C_8$, cycloalkyle en $C_4$-$C_8$, cyano(alkyle en $C_2$-$C_4$), hydroxyalkyle en $C_2$-$C_4$, chloroalkyle en $C_2$-$C_4$, phényl(alkyle en $C_1$-$C_2$) éventuellement substitué par un atome de chlore ou phényle éventuellement substitué par un atome de chlore ou par un radical méthyle, ou bien $R^1$ et $R^2$ forment ensemble, avec l'atome d'azote qui les relie, un reste hétérocyclique saturé à 5 ou 6 termes qui peut contenir d'autres hétéroatomes,

$R^3$ représente un atome d'hydrogène, de fluor, de chlore ou un reste alkyle en $C_1$-$C_2$ ou alkoxy en $C_1$-$C_5$, ou bien, lorsque $R^3$ est en position ortho par rapport au reste -$NR^1R^2$, $R^3$ et $R^2$ forment ensemble un reste alkylène en $C_2$-$C_4$, qui peut être substitué par 1 à 3 radicaux alkyle en $C_1$-$C_4$, ou un reste 1,2-phénylène, et

A représente un reste naphtyle ou un reste de formule II

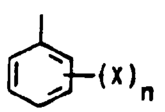

(II),

dans laquelle

X est mis pour un atome d'hydrogène, de fluor, de chlore, de brome ou pour un reste alkyle en $C_1$-$C_5$, hydroxy, alcoxy en $C_1$-$C_5$, phénoxy, phényl(alcoxy en $C_1$-$C_2$), cyano ou phényle et

n est mis pour 1 ou 2,

étant spécifié que $R^1$ et $R^2$ ne sont pas mis simultanément pour un reste méthyle lorsque A a la signification d'un reste phényle.

2. 3-Aminophénylpentane-1,5-diones selon la revendication 1, caractérisées en ce que A représente un reste naphtyle-1, napthyle-2 ou un reste de formule II

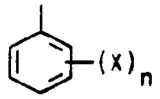

(II),

dans laquelle

X est mis pour un atome de fluor, de chlore, de brome ou pour un reste alkyle en $C_1$-$C_5$, hydroxy, alcoxy en $C_1$-$C_5$, phénoxy, phényl(alcoxy en $C_1$-$C_2$), cyano ou phényle, et

n est mis pour 1 ou 2.

**3.** 3-Aminophénylpentane-1,5-diones selon la revendication 1, caractérisées en ce que $R^1$ et $R^2$ représentent chacun, indépendamment l'un de l'autre, un reste alkyle en $C_1$-$C_4$ non ramifié, cyanoéthyle ou benzyle et $R^3$ représente un atome d'hydrogène.

**4.** 3-Aminophénylpentane-1,5-diones selon la revendication 1, caractérisées en ce que A représente un reste naphtyle-1, naphtyle-2 ou un reste de formule IIa

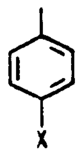

(IIa),

dans laquelle

X est mis pour un atome de fluor, de chlore ou pour un reste alkyle en $C_1$-$C_5$, alcoxy en $C_1$-$C_5$, cyano ou phényle.

**5.** 3-Aminophénylpentane-1,5-diones selon la revendication 1, caractérisées en ce que $R^1$ et $R^2$ représentent chacun, indépendamment l'un de l'autre, un reste alkyle en $C_1$-$C_4$ non ramifié, cyanoéthyle ou benzyle et A représente chaque fois un reste 4-chlorophényle.

**6.** 3-Aminophénylpentane-1,5-diones selon la revendication 1, caractérisées en ce que $R^1$ et $R^2$ représentent chacun un reste méthyle.